# EUROPEAN PATENT SPECIFICATION

(11) **EP 3 178 847 B1**
(45) Date of publication and mention of the grant of the patent: **03.03.2021**
(21) Application number: 15830493.1
(22) Date of filing: 18.03.2015
(51) Int. Cl.: C07K 16/24, C12N 15/13, A61K 39/395, A61P 35/00, A61P 29/00, A61P 19/02, A61P 37/02, G01N 33/68, G01N 33/577

(54) **ANTI-TNF-ALPHA FULLY HUMAN MONOCLONAL ANTIBODIES WITH LOW IMMUNOGENICITY AND APPLICATION THEREOF**
VOLLSTÄNDIG HUMANE MONOKLONALE ANTI-TNF-ALPHA-ANTIKÖRPER MIT NIEDRIGER IMMUNOGENITÄT UND ANWENDUNG DAVON
ANTICORPS MONOCLONAUX ANTI-TNF-ALPHA ENTIÈREMENT HUMAINS À FAIBLE IMMUNOGÉNICITÉ ET APPLICATION ASSOCIÉE

(30) Priority: 08.08.2014 CN 201410390493
(43) Date of publication of application: 14.06.2017
(73) Proprietor: Abmax Biotechnology Co., Ltd, Beijing 101111 (CN)
(72) Inventor: SUN, Le, Beijing 101111 (CN); ZHANG, Xiaogang, Beijing 101111 (CN); LI, Maohua, Beijing 101111 (CN); ZHANG, Cuijuan, Beijing 101111 (CN)
(74) Representative: Potter Clarkson
(86) International application number: PCT/CN2015/074528
(87) International publication number: WO 2016/019726

(56) References cited:
- WO-A1-98/52976
- WO-A1-2009/083246
- WO-A1-2010/121140
- CN-A- 102 439 040
- CN-A- 102 755 646
- CN-A- 104 341 502
- HARDING FIONA A ET AL: "The immunogenicity of humanized and fully human antibodies: Residual immunogenicity resides in the CDR regions", 20100501, vol. 2, no. 3, 1 May 2010 (2010-05-01), pages 256-265, XP009137415, ISSN: 1942-0870, DOI: 10.4161/MABS.2.3.11641
- NIKO K BENDER ET AL: "Immunogenicity, efficacy and adverse events of adalimumab in RA patients", RHEUMATOLOGY INTERNATIONAL ; CLINICAL AND EXPERIMENTAL INVESTIGATIONS, SPRINGER, BERLIN, DE, vol. 27, no. 3, 28 September 2006 (2006-09-28), pages 269-274, XP019473129, ISSN: 1437-160X, DOI: 10.1007/S00296-006-0183-7
- R. L. WEST ET AL: "Immunogenicity negatively influences the outcome of adalimumab treatment in Crohn's disease", ALIMENTARY PHARMACOLOGY & THERAPEUTICS., vol. 28, no. 9, 1 November 2008 (2008-11-01), pages 1122-1126, XP055414496, GB ISSN: 0269-2813, DOI: 10.1111/j.1365-2036.2008.03828.x
- PAULINE A. VAN SCHOUWENBURG ET AL: "Functional Analysis of the Anti-adalimumab Response Using Patient-derived Monoclonal Antibodies", JOURNAL OF BIOLOGICAL CHEMISTRY, vol. 289, no. 50, 17 October 2014 (2014-10-17), pages 34482-34488, XP055414609, ISSN: 0021-9258, DOI: 10.1074/jbc.M114.615500

### SEQUENCE LISTING

<110> Abmax Biotechnology Co., Ltd.
   <120> ANTI-TNF-alpha FULLY HUMAN MONOCLONAL ANTIBODIES WITH LOW IMMUNOGENICITY AND APPLICATION THEREOF
<130> CNKBP/P62868EP
<140> EP 15830493.1
   <141> 2015-03-18
<150> PCT/CN2015/074528
   <151> 2015-03-18
<150> CN 201410390493.4
   <151> 2014-08-08
<160> 24
<170> PatentIn version 3.5
<210> 1
   <211> 714
   <212> DNA
   <213> Modified light chain L1 of human anti-TNF-alpha antibody
<400> 1
   atggacatga gggtccctgc tcagctcctg ggactcctgc tgctctggct cccagatacc 60
   agatgtgaca tccagatgac ccagtctcca tcctccctgt ctgcatctgt aggagacaga 120
   gtcaccatca cttgccgggc gagtcagggc attaggaatt atttagcctg gtatcagcag 180
   aaaccaggga aagctcctaa actcctgatc tatgctgcat ccactttgca atcaggggtc 240
   ccatctcggt tcagcggcag tggatctggg acaggtttca ctctcaccat cagcagcctg 300
   cagcctgaag atgttgcaac ttattactgt caaaggtata acagagcccc gtacactttt 360
   ggccagggga ccaaggtgga gatcaaacga actgtggctg caccatctgt cttcatcttc 420
   ccgccatctg atgagcagtt gaaatctgga actgcctctg ttgtgtgcct gctgaataac 480
   ttctatccca gagaggccaa agtacagtgg aaggtggata acgccctcca atcgggtaac 540
   tcccaggaga gtgtcacaga gcaggacagc aaggacagca cctacagcct cagcagcacc 600
   ctgacgctga gcaaagcaga ctacgagaaa cacaaagtct acgcctgcga agtcacccat 660
   cagggcctga gctcgcccgt cacaaagagc ttcaacaggg gagagtgtta gtaa 714
<210> 2
   <211> 714
   <212> DNA
   <213> Modified light chain L2 of human anti-TNF-alpha antibody
<400> 2
   atggacatga gggtccctgc tcagctcctg ggactcctgc tgctctggct cccagatacc 60
   agatgtgaca tccagatgac ccagtctcca tcctccctgt ctgcatctgt aggagacaga 120
   gtcaccatca cttgccgggc gagtcagggc attaggaatt atttagcctg gtatcagcag 180
   aaaccaggga aagctcctaa actcctgatc tatgctgcat ccactttgca atcaggggtc 240
   ccatctcggt tcagcggcag tggatctggg acaggtttca ctctcaccat cagcagcctg 300
   cagcctgaag attttgcaac ttattactgt caaaggtata acagagcccc gtacactttt 360
   ggccagggga ccaaggtgga gatcaaacga actgtggctg caccatctgt cttcatcttc 420
   ccgccatctg atgagcagtt gaaatctgga actgcctctg ttgtgtgcct gctgaataac 480
   ttctatccca gagaggccaa agtacagtgg aaggtggata acgccctcca atcgggtaac 540
   tcccaggaga gtgtcacaga gcaggacagc aaggacagca cctacagcct cagcagcacc 600
   ctgacgctga gcaaagcaga ctacgagaaa cacaaagtct acgcctgcga agtcacccat 660
   cagggcctga gctcgcccgt cacaaagagc ttcaacaggg gagagtgtta gtaa 714
<210> 3
   <211> 714
   <212> DNA
   <213> Modified light chain L3 of human anti-TNF-alpha antibody
<400> 3
   atggacatga gggtccctgc tcagctcctg ggactcctgc tgctctggct cccagatacc 60
   agatgtgaca tccagatgac ccagtctcca tcctccgtgt ctgcatctgt aggagacaga 120
   gtcaccatca cttgccgggc gagtcagggc attaggaatt atttagcctg gtatcagcag 180
   aaaccaggga aagctcctaa actcctgatc tatgctgcat ccactttgca atcaggggtc 240
   ccatctcggt tcagcggcag tggatctggg acaggtttca ctctcaccat cagcagcctg 300
   cagcctgaag atgttgcaac ttattactgt caaaggtata acagagcccc gtacactttt 360
   ggccagggga ccaaggtgga gatcaaacga actgtggctg caccatctgt cttcatcttc 420
   ccgccatctg atgagcagtt gaaatctgga actgcctctg ttgtgtgcct gctgaataac 480
   ttctatccca gagaggccaa agtacagtgg aaggtggata acgccctcca atcgggtaac 540
   tcccaggaga gtgtcacaga gcaggacagc aaggacagca cctacagcct cagcagcacc 600
   ctgacgctga gcaaagcaga ctacgagaaa cacaaagtct acgcctgcga agtcacccat 660
   cagggcctga gctcgcccgt cacaaagagc ttcaacaggg gagagtgtta gtaa 714
<210> 4
   <211> 714
   <212> DNA
   <213> Modified light chain L4 of human anti-TNF-alpha antibody
<400> 4
   atggacatga gggtccctgc tcagctcctg ggactcctgc tgctctggct cccagatacc      60
   agatgtgaca tccagatgac ccagtctcca tcctccgtgt ctgcatctgt aggagacaga     120
   gtcaccatca cttgccgggc gagtcagggc attaggaatt atttagcctg gtatcagcag     180
   aaaccaggga aagctcctaa actcctgatc tatgctgcat ccactttgca atcaggggtc     240
   ccatctcggt tcagcggcag tggatctggg acaggtttca ctctcaccat cagcagcctg     300
   cagcctgaag attttgcaac ttattactgt caaaggtata acagagcccc gtacactttt     360
   ggccagggga ccaaggtgga gatcaaacga actgtggctg caccatctgt cttcatcttc     420
   ccgccatctg atgagcagtt gaaatctgga actgcctctg ttgtgtgcct gctgaataac     480
   ttctatccca gagaggccaa agtacagtgg aaggtggata acgccctcca atcgggtaac     540
   tcccaggaga gtgtcacaga gcaggacagc aaggacagca cctacagcct cagcagcacc     600
   ctgacgctga gcaaagcaga ctacgagaaa cacaaagtct acgcctgcga agtcacccat     660
   cagggcctga gctcgcccgt cacaaagagc ttcaacaggg gagagtgtta gtaa           714
<210> 5
   <211> 714
   <212> DNA
   <213> Modified light chain L5 of human anti-TNF-alpha antibody
<400> 5
   atggacatga gggtccctgc tcagctcctg ggactcctgc tgctctggct cccagatacc      60
   agatgtgaca tccagatgac ccagtctcca tcctccgtgt ctgcatctgt aggagacaga     120
   gtcaccatca cttgccgggc gagtcagggc attaggaatt atttagcctg gtatcagcag     180
   aaaccaggga aagtgcctgt actcctgatc tatgctgcat ccactttgca atcaggggtc     240
   ccatctcggt tcagcggcag tggatctggg acaggtttca ctctcaccat cagcagcctg     300
   cagcctgaag atgttgcaac ttattactgt caaaggtata acagagcccc gtacactttt     360
   ggccagggga ccaaggtgga gatcaaacga actgtggctg caccatctgt cttcatcttc     420
   ccgccatctg atgagcagtt gaaatctgga actgcctctg ttgtgtgcct gctgaataac     480
   ttctatccca gagaggccaa agtacagtgg aaggtggata acgccctcca atcgggtaac     540
   tcccaggaga gtgtcacaga gcaggacagc aaggacagca cctacagcct cagcagcacc     600
   ctgacgctga gcaaagcaga ctacgagaaa cacaaagtct acgcctgcga agtcacccat     660
   cagggcctga gctcgcccgt cacaaagagc ttcaacaggg gagagtgtta gtaa           714
<210> 6
   <211> 522
   <212> DNA
   <213> Modified heavy chain H1 of human anti-TNF-alpha antibody
<400> 6
   atggagttgg gactgagctg gattttcctt ttggctattt taaaaggtgt ccagtgtgag      60
   gtgcagctgg tggagtctgg gggaggcttg gtacagcccg gcaggtccct gagactctcc     120
   tgtgcggcct ctggattcac ctttgatgat tatgccatgc actgggtccg gcaagctcca     180
   gggaagggcc tggaatgggt ctcagctatc acttggaata gtggtcacat agactatgcg     240
   gactctgtgg agggccgatt caccatctcc ctggacacct ccaagtccac cgcttatctg     300
   caaatgaaca gtctgagagc tgaggattca gccgtatatt actgtgcgaa agtctcgtac     360
   cttagcaccg cgtcctccct tgactattgg ggccaaggaa ccctggtcac cgtctcgagt     420
   gcctccacca agggcccatc ggtcttcccc ctggcaccct cctccaagag cacctctggg     480
   ggcacagcag ccctgggctg cctggtcaag gactacttcc cc                        522
<210> 7
   <211> 522
   <212> DNA
   <213> Modified heavy chain H2 of human anti-TNF-alpha antibody
<400> 7
   atggagttgg gactgagctg gattttcctt ttggctattt taaaaggtgt ccagtgtgag     60
   gtgcagctgg tggagtctgg gggaggcttg gtacagcccg gcaggtccct gagactctcc    120
   tgtgcggcct ctggattcac ctttgatgat tatgccatgc actgggtccg gcaagctcca    180
   gggaagggcc tggaatgggt ctcagctatc acttggaata gtggtcacat agactatgcg    240
   gactctgtgg agggccgatt caccttctcc ctggacacct ccaagtccac cgcttatctg    300
   caaatgaaca gtctgagagc tgaggatacg gccgtatatt actgtgcgaa agtctcgtac    360
   cttagcaccg cgtcctccct tgactattgg ggccaaggaa ccctggtcac cgtctcgagt    420
   gcctccacca agggcccatc ggtcttcccc ctggcaccct cctccaagag cacctctggg    480
   ggcacagcag ccctgggctg cctggtcaag gactacttcc cc                       522
<210> 8
   <211> 522
   <212> DNA
   <213> Modified heavy chain H3 of human anti-TNF-alpha antibody
<400> 8
   atggagttgg gactgagctg gattttcctt ttggctattt taaaaggtgt ccagtgtgag     60
   gtgcagctgg tggagtctgg gggaggcttg gtacagcccg gcggatccct gagactctcc    120
   tgtgcggcct ctggatacac ctttgatgat tatgccatgc actgggtccg gcaagctcca    180
   gggaagggcc tggaatgggt ctcagctatc acttggaata gtggtcacat agactatgcg    240
   gactctgtgg agggccgatt caccatctcc ctggacacct ccaagtccac cgcttatctg    300
   caaatgaaca gtctgagagc tgaggattca gccgtatatt actgtgcgaa agtctcgtac    360
   cttagcaccg cgtcctccct tgactattgg ggccaaggaa ccctggtcac cgtctcgagt    420
   gcctccacca agggcccatc ggtcttcccc ctggcaccct cctccaagag cacctctggg    480
   ggcacagcag ccctgggctg cctggtcaag gactacttcc cc                       522
<210> 9
   <211> 522
   <212> DNA
   <213> Modified heavy chain H4 of human anti-TNF-alpha antibody
<400> 9
   atggagttgg gactgagctg gattttcctt ttggctattt taaaaggtgt ccagtgtgag     60
   gtgcagctgg tggagtctgg gggaggcttg gtacagcccg gcggatccct gagactctcc    120
   tgtgcggcct ctggatacac ctttgatgat tatgccatgc actgggtccg gcaagctcca    180
   gggaagggcc tggaatgggt ctcagctatc acttggaata gtggtcacat agactatgcg    240
   gactctgtgg agggccgatt caccttctcc ctggacacct ccaagtccac cgcttatctg    300
   caaatgaaca gtctgagagc tgaggatacg gccgtatatt actgtgcgaa agtctcgtac    360
   cttagcaccg cgtcctccct tgactattgg ggccaaggaa ccctggtcac cgtctcgagt    420
   gcctccacca agggcccatc ggtcttcccc ctggcaccct cctccaagag cacctctggg    480
   ggcacagcag ccctgggctg cctggtcaag gactacttcc cc                       522
<210> 10
   <211> 522
   <212> DNA
   <213> Modified heavy chain H5 of human anti-TNF-alpha antibody
<400> 10
   atggagttgg gactgagctg gattttcctt ttggctattt taaaaggtgt ccagtgtgag     60
   gtgcagctgg tggagtctgg gggaggcttg gtacagcccg gcggatccct gagactctcc    120
   tgtgcggcct ctggatacac ctttgatgat tatgccatgc actgggtccg gcaagctcca    180
   gggaagggcc tggaatgggt ctcagctatc acttggaata gtggtcacat agactatgcg    240
   gactctgtgg agggccgatt caccatctcc ctggacacct ccgtatccac cgcttatctg    300
   caaatgaaca gtctgagagc tgaggattca gccgtatatt actgtgcgaa agtctcgtac    360
   cttagcaccg cgtcctccct tgactattgg ggccaaggaa ccctggtcac cgtctcgagt    420
   gcctccacca agggcccatc ggtcttcccc ctggcaccct cctccaagag cacctctggg    480
   ggcacagcag ccctgggctg cctggtcaag gactacttcc cc                       522
<210> 11
   <211> 236
   <212> PRT
<210> 12
   <211> 236
   <212> PRT
   <213> Modified light chain L2 of human anti-TNF-alpha antibody
<400> 12
   Met Asp Met Arg Val Pro Ala Gln Leu Leu Gly Leu Leu Leu Leu Trp
   1               5                   10                  15
   Leu Pro Asp Thr Arg Cys Asp Ile Gln Met Thr Gln Ser Pro Ser Ser
               20                  25                  30
   Leu Ser Ala Ser Val Gly Asp Arg Val Thr Ile Thr Cys Arg Ala Ser
           35                  40                  45
   Gln Gly Ile Arg Asn Tyr Leu Ala Trp Tyr Gln Gln Lys Pro Gly Lys
       50                  55                  60
   Ala Pro Lys Leu Leu Ile Tyr Ala Ala Ser Thr Leu Gln Ser Gly Val
   65                  70                  75                  80
   Pro Ser Arg Phe Ser Gly Ser Gly Ser Gly Thr Gly Phe Thr Leu Thr
                   85                  90                  95
   Ile Ser Ser Leu Gln Pro Glu Asp Phe Ala Thr Tyr Tyr Cys Gln Arg
               100                 105                 110
   Tyr Asn Arg Ala Pro Tyr Thr Phe Gly Gln Gly Thr Lys Val Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 13
   <211> 236
   <212> PRT
   <213> Modified light chain L3 of human anti-TNF-alpha antibody
<400> 13
   Met Asp Met Arg Val Pro Ala Gln Leu Leu Gly Leu Leu Leu Leu Trp
   1               5                   10                  15
   Leu Pro Asp Thr Arg Cys Asp Ile Gln Met Thr Gln Ser Pro Ser Ser
               20                  25                  30
   Val Ser Ala Ser Val Gly Asp Arg Val Thr Ile Thr Cys Arg Ala Ser
           35                  40                  45
   Gln Gly Ile Arg Asn Tyr Leu Ala Trp Tyr Gln Gln Lys Pro Gly Lys
       50                  55                  60
   Ala Pro Lys Leu Leu Ile Tyr Ala Ala Ser Thr Leu Gln Ser Gly Val
   65                  70                  75                  80
   Pro Ser Arg Phe Ser Gly Ser Gly Ser Gly Thr Gly Phe Thr Leu Thr
                   85                  90                  95
   Ile Ser Ser Leu Gln Pro Glu Asp Val Ala Thr Tyr Tyr Cys Gln Arg
               100                 105                 110
   Tyr Asn Arg Ala Pro Tyr Thr Phe Gly Gln Gly Thr Lys Val Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 14
   <211> 236
   <212> PRT
   <213> Modified light chain L4 of human anti-TNF-alpha antibody
<400> 14
   Met Asp Met Arg Val Pro Ala Gln Leu Leu Gly Leu Leu Leu Leu Trp
   1               5                   10                  15
   Leu Pro Asp Thr Arg Cys Asp Ile Gln Met Thr Gln Ser Pro Ser Ser
               20                  25                  30
   Val Ser Ala Ser Val Gly Asp Arg Val Thr Ile Thr Cys Arg Ala Ser
           35                  40                  45
   Gln Gly Ile Arg Asn Tyr Leu Ala Trp Tyr Gln Gln Lys Pro Gly Lys
       50                  55                  60
   Ala Pro Lys Leu Leu Ile Tyr Ala Ala Ser Thr Leu Gln Ser Gly Val
   65                  70                  75                  80
   Pro Ser Arg Phe Ser Gly Ser Gly Ser Gly Thr Gly Phe Thr Leu Thr
                   85                  90                  95
   Ile Ser Ser Leu Gln Pro Glu Asp Phe Ala Thr Tyr Tyr Cys Gln Arg
               100                 105                 110
   Tyr Asn Arg Ala Pro Tyr Thr Phe Gly Gln Gly Thr Lys Val Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 15
   <211> 236
   <212> PRT
   <213> Modified light chain L5 of human anti-TNF-alpha antibody
<400> 15
   Met Asp Met Arg Val Pro Ala Gln Leu Leu Gly Leu Leu Leu Leu Trp
   1               5                   10                  15
   Leu Pro Asp Thr Arg Cys Asp Ile Gln Met Thr Gln Ser Pro Ser Ser
               20                  25                  30
   Val Ser Ala Ser Val Gly Asp Arg Val Thr Ile Thr Cys Arg Ala Ser
           35                  40                  45
   Gln Gly Ile Arg Asn Tyr Leu Ala Trp Tyr Gln Gln Lys Pro Gly Lys
       50                  55                  60
   Val Pro Val Leu Leu Ile Tyr Ala Ala Ser Thr Leu Gln Ser Gly Val
   65                  70                  75                  80
   Pro Ser Arg Phe Ser Gly Ser Gly Ser Gly Thr Gly Phe Thr Leu Thr
                   85                  90                  95
   Ile Ser Ser Leu Gln Pro Glu Asp Val Ala Thr Tyr Tyr Cys Gln Arg
               100                 105                 110
   Tyr Asn Arg Ala Pro Tyr Thr Phe Gly Gln Gly Thr Lys Val Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 16
   <211> 174
   <212> PRT
   <213> Modified heavy chain h1 of human anti-TNF-alpha antibody
<400> 16
   Met Glu Leu Gly Leu Ser Trp Ile Phe Leu Leu Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Arg Ser Leu Arg Leu Ser Cys Ala Ala Ser Gly Phe Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Ile Ser Leu Asp Thr Ser Lys Ser
                   85                  90                  95
   Thr Ala Tyr Leu Gln Met Asn Ser Leu Arg Ala Glu Asp Ser Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro
                   165                 170
<210> 17
   <211> 174
   <212> PRT
   <213> Modified heavy chain h2 of human anti-TNF-alpha antibody
<400> 17
   Met Glu Leu Gly Leu Ser Trp Ile Phe Leu Leu Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Arg Ser Leu Arg Leu Ser Cys Ala Ala Ser Gly Phe Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Phe Ser Leu Asp Thr Ser Lys Ser
                   85                  90                  95
   Thr Ala Tyr Leu Gln Met Asn Ser Leu Arg Ala Glu Asp Thr Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro
                   165                 170
<210> 18
   <211> 174
   <212> PRT
   <213> Modified heavy chain h3 of human anti-TNF-alpha antibody
<400> 18
   Met Glu Leu Gly Leu Ser Trp Ile Phe Leu Leu Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Gly Ser Leu Arg Leu Ser Cys Ala Ala Ser Gly Tyr Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Ile Ser Leu Asp Thr Ser Lys Ser
                   85                  90                  95
   Thr Ala Tyr Leu Gln Met Asn Ser Leu Arg Ala Glu Asp Ser Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro
                   165                 170
<210> 19
   <211> 174
   <212> PRT
   <213> Modified heavy chain h4 of human anti-TNF-alpha antibody
<400> 19
   Met Glu Leu Gly Leu Ser Trp Ile Phe Leu Leu Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Gly Ser Leu Arg Leu Ser Cys Ala Ala Ser Gly Tyr Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Phe Ser Leu Asp Thr Ser Lys Ser
                   85                  90                  95
   Thr Ala Tyr Leu Gln Met Asn Ser Leu Arg Ala Glu Asp Thr Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro
                   165                 170
<210> 20
   <211> 174
   <212> PRT
   <213> Modified heavy chain h5 of human anti-TNF-alpha antibody
<400> 20
   Met Glu Leu Gly Leu Ser Trp Ile Phe Leu Leu Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Gly Ser Leu Arg Leu Ser Cys Ala Ala Ser Gly Tyr Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Ile Ser Leu Asp Thr Ser Val Ser
                   85                  90                  95
   Thr Ala Tyr Leu Gln Met Asn Ser Leu Arg Ala Glu Asp Ser Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro
                   165                 170
<210> 21
   <211> 714
   <212> DNA
   <213> Light chain L0 of Adalimumab
<400> 21
   atggacatga gggtccctgc tcagctcctg ggactcctgc tgctctggct cccagatacc      60
   agatgtgaca tccagatgac ccagtctcca tcctccctgt ctgcatctgt aggagacaga     120
   gtcaccatca cttgccgggc gagtcagggc attaggaatt atttagcctg gtatcagcag     180
   aaaccaggga aagctcctaa actcctgatc tatgctgcat ccactttgca atcaggggtc     240
   ccatctcggt tcagcggcag tggatctggg acagatttca ctctcaccat cagcagcctg     300
   cagcctgaag atgttgcaac ttattactgt caaaggtata acagagcccc gtacactttt     360
   ggccagggga ccaaggtgga gatcaaacga actgtggctg caccatctgt cttcatcttc     420
   ccgccatctg atgagcagtt gaaatctgga actgcctctg ttgtgtgcct gctgaataac     480
   ttctatccca gagaggccaa agtacagtgg aaggtggata acgccctcca atcgggtaac     540
   tcccaggaga gtgtcacaga gcaggacagc aaggacagca cctacagcct cagcagcacc     600
   ctgacgctga gcaaagcaga ctacgagaaa cacaaagtct acgcctgcga agtcacccat     660
   cagggcctga gctcgcccgt cacaaagagc ttcaacaggg gagagtgtta gtaa           714
<210> 22
   <211> 522
   <212> DNA
   <213> Heavy chain h0 of Adalimumab
<400> 22
   atggagttgg gactgagctg gattttcctt ttggctattt taaaaggtgt ccagtgtgag      60
   gtgcagctgg tggagtctgg gggaggcttg gtacagcccg gcaggtccct gagactctcc     120
   tgtgcggcct ctggattcac ctttgatgat tatgccatgc actgggtccg gcaagctcca     180
   gggaagggcc tggaatgggt ctcagctatc acttggaata gtggtcacat agactatgcg     240
   gactctgtgg agggccgatt caccatctcc agagacaacg ccaagaactc cctgtatctg     300
   caaatgaaca gtctgagagc tgaggatacg gccgtatatt actgtgcgaa agtctcgtac     360
   cttagcaccg cgtcctccct tgactattgg ggccaaggaa ccctggtcac cgtctcgagt     420
   gcctccacca agggcccatc ggtcttcccc ctggcaccct cctccaagag cacctctggg     480
   ggcacagcag ccctgggctg cctggtcaag gactacttcc cc                        522
<210> 23
   <211> 236
   <212> PRT
   <213> Light chain L0 of Adalimumab
<400> 23
   Met Asp Met Arg Val Pro Ala Gln Leu Leu Gly Leu Leu Leu Leu Trp
   1               5                   10                  15
   Leu Pro Asp Thr Arg Cys Asp Ile Gln Met Thr Gln Ser Pro Ser Ser
               20                  25                  30
   Leu Ser Ala Ser Val Gly Asp Arg Val Thr Ile Thr Cys Arg Ala Ser
           35                  40                  45
   Gln Gly Ile Arg Asn Tyr Leu Ala Trp Tyr Gln Gln Lys Pro Gly Lys
       50                  55                  60
   Ala Pro Lys Leu Leu Ile Tyr Ala Ala Ser Thr Leu Gln Ser Gly Val
   65                  70                  75                  80
   Pro Ser Arg Phe Ser Gly Ser Gly Ser Gly Thr Asp Phe Thr Leu Thr
                   85                  90                  95
   Ile Ser Ser Leu Gln Pro Glu Asp Val Ala Thr Tyr Tyr Cys Gln Arg
               100                 105                 110
   Tyr Asn Arg Ala Pro Tyr Thr Phe Gly Gln Gly Thr Lys Val Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 24
   <211> 174
   <212> PRT
   <213> Heavy chain h0 of Adalimumab
<400> 24
   Met Glu Leu Gly Leu Ser Trp Ile Phe Leu Leu Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Arg Ser Leu Arg Leu Ser Cys Ala Ala Ser Gly Phe Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro
                   165                 170

## Description

### TECHNICAL FIELD

The present invention relates to de-immunogenicity of anti-Tumor Necrosis Factor-alpha (TNF-α) antibodies and applications of using the same for treating inflammatory diseases and other human diseases.

### BACKGROUND

TNF is an immunity-modulating cytokine required for immune processes. The unregulated activities of TNFs can lead to the development of inflammatory diseases. Excess amounts of TNF-expressed in cells are associated with the development of immune diseases, including rheumatoid arthritis, Crohn's disease, psoriatic arthritis, and inflammatory bowel disease. The function of TNF requires binding to its two receptors, TNF receptor 1 (TNFR1) and TNF receptor 2 (TNFR2). Blocking the interaction between TNF and TNFRs has successfully been developed as a therapy in treating inflammatory or autoimmune diseases. TNF neutralization therapies, including the use of a soluble TNFR2-Fc recombinant (Etanercept), a mouse-human chimera mAb (Infliximab), or a human mAb (Adalimumab), have been introduced in the past decades for the management of rheumatoid arthritis and other immune diseases.

However, although it is fully human antibody, high immunogenicity has been observed in human patients treated with Adalimumab. Anti-drug antibody (ADA) to Adalimumab was detected in up to 75% of the patients. It was also reported that the annual loss of response to Adalimumab was calculated to be 24%. ADA was considered as the causes of treatment failures, and it is believed that ADAs might reduce drug efficacy by competing with the endogenous ligand (neutralizing antibodies, Nab) and/or by forming immune complex, which accelerate the clearance of the drug from the circulation. Therefore there is need to develop a better anti-TNF antibody with lower immunogenicity and longer efficicay.

This invention is about the de-immunogenicity of human anti-Tumor Necrosis Factor-alpha (TNF-α) antibody Adalimumab, designed as clones TCX002-L3H4, L1H4. etc, which bind to the same epitope from the one recognized by Adalimumab, but with much lower immunogenicities in vivo.

WO 2010/121140 (Abbott Biotherapeutics Corp. et al.) relates to anti-TNF-a antibodies and their uses.

WO 98/52976 (Biovation Limited et al.) relates to a method for the production of non-immunogenic proteins.

Harding et al. (MAbs 2010, 2(3):256-65) relates to the immunogenicity of humanized and fully human antibodies.

Bender et al. (Rheumatol. Int. 2007, 27(3):269-74) relates to immunogenicity, efficacy and adverse events of adalimumab in rheumatoid arthritis patients.

West et al. (Aliment. Pharmacol. Ther. 2008, 28(9): 1122-6) relates to how immunogenicity negatively infuences the outcome of adalimumab treatment in Crohn's disease.

van Schouwenburg et al. (J. Biol. Chem. 2014, 289(50):34482-8) relates to functional analysis of the anti-adalimumab response using patient-derived monoclonal antibodies.

WO 2009/083246 (Bayer Schering Pharma Aktiengesellschaft et al.) relates to antibodies to TNF-α.

### SUMMARY OF THE INVENTION

The present invention is as defined in the claims and relates to a low immunogenic human anti-TNF-a antibody comprising the CDR regions of heavy (SEQ ID NO. 23) and light (SEQ ID NO. 24) chains from human Adalimumab, but modification/replacement of amino acid sequence(s) in the FR regions of Adalimumab, wherein the antibody has reduced immunogenicity compared to Adalimumab, and wherein the antibody comprises:
(a) the human light chain amino acid sequence of SEQ ID NO: 13 and the human heavy chain amino acid sequence of SEQ ID NO: 17; or
(b) the human light chain amino acid sequence of SEQ ID NO: 13 and the human heavy chain amino acid sequence of SEQ ID NO: 19; or
(c) the human light chain amino acid sequence of SEQ ID NO: 14 and the human heavy chain amino acid sequence of SEQ ID NO: 16; or
(d) the human light chain amino acid sequence of SEQ ID NO: 14 and the human heavy chain amino acid sequence of SEQ ID NO: 17; or
(e) the human light chain amino acid sequence of SEQ ID NO: 14 and the human heavy chain amino acid sequence of SEQ ID NO: 19; or
(f) the human light chain amino acid sequence of SEQ ID NO: 12 and the human heavy chain amino acid sequence of SEQ ID NO: 16; or
(g) the human light chain amino acid sequence of SEQ ID NO: 23 and the human heavy chain amino acid sequence of SEQ ID NO: 19.

To the extent that other low immunogenic human anti-TNF-α antibodies are disclosed herein, they are included merely for reference purposes.

The present invention provides the human anti-Tumor Necrosis Factor-alpha (TNF-α) antibodies with reduced immunogenicities and methods of using the same for neutralizing the TNF-α induced cell death and for treating inflammatory diseases and other human diseases. In one aspect, the present invention features TNF-α-binding molecules and their DNA and amino acid sequences. Each molecule comprises the CDRs from human anti-TNF-α monoclonal antibody Adalimumab and the FRs from different human origins.

Also described is a method to develop human antibodies with reduced immunogenicities by replacing the FRs of the original human monoclonal antibody with the FRs from different human origins.

Also described is one example of using the method to develop human anti-TNF-α antibodies with reduced immunogenicities by replacing the FRs of human anti-TNF-α monoclonal antibody Adalimumab with the FRs from different human origins.

Described are de-immunized human anti-TNF-α antibodies with one of the amino acid sequences of light chains shown in SEQ ID NO.11∼15 or 23, and one of the amino acid sequences of heavy chains shown in SEQ ID NO.16∼20.

Described are de-immunized human anti-TNF-a antibodies with one of the DNA sequences of light chains L1-L5 shown in SEQ ID NO.1∼5, and one of the DNA sequences of heavy chains hl-h5 shown in SEQ ID NO.6∼10.

Furthermore, the present invention features de-immunized human anti-TNF-a antibody with the the amino acid sequence of light chains shown in SEQ ID NO.13, and the amino acid sequences of heavy chain shown in SEQ ID 19.

Furthermore, the present invention features de-immunized human anti-TNF-a antibody with the DNA sequence of light chain shown in SEQ ID NO.3, and the DNA sequence of heavy chain shown in SEQ ID.9

Provided are the sequences for 10 de-immunized human anti-TNF-a antibodies, named as L3h2, L3h4, L5h2, L4h1, L4h2, L4h4, L1h3, L2h1, L0h4 and L2h5, which have similar affinities as the original and can block the binding of TNF-α to its receptors TNFRs p55 and p75. The antibodies of the present invention are L3h2, L3h4, L4h1, L4h2, L4h4, L2h1 and L0h4.

Whereas, the de-immunized human anti-TNF-α antibody L3h2 with the amino acid sequence of light chains shown in SEQ ID NO.13 and the amino acid sequences of heavy chain shown in SEQ ID No.17, and with the DNA sequence of light chain shown in SEQ ID NO.3, and the DNA sequence of heavy chain shown in SEQ ID No.7.

Whereas, the de-immunized human anti-TNF-α antibody L3h4 with the amino acid sequence of light chains shown in SEQ ID NO.13 and the amino acid sequences of heavy chain shown in SEQ ID No.19 and with the DNA sequence of light chain shown in SEQ ID NO.3, and the DNA sequence of heavy chain shown in SEQ ID No.9.

Whereas, the de-immunized human anti-TNF-α antibody L5h2 with the amino acid sequence of light chains shown in SEQ ID NO.15 and the amino acid sequences of heavy chain shown in SEQ ID No.17, and with the DNA sequence of light chain shown in SEQ ID NO.5, and the DNA sequence of heavy chain shown in SEQ ID No.7.

Whereas, the de-immunized human anti-TNF-α antibody L4h1 with the amino acid sequence of light chains shown in SEQ ID NO.14 and the amino acid sequences of heavy chain shown in SEQ ID No.16, and with the DNA sequence of light chain shown in SEQ ID NO.4, and the DNA sequence of heavy chain shown in SEQ ID No.6.

Whereas, the de-immunized human anti-TNF-a antibody L4h2 with the amino acid sequence of light chains shown in SEQ ID NO.14 and the amino acid sequences of heavy chain shown in SEQ ID No.17, and with the DNA sequence of light chain shown in SEQ ID NO.4, and the DNA sequence of heavy chain shown in SEQ ID No.7.

Whereas, the de-immunized human anti-TNF-α antibody L4h4 with the amino acid sequence of light chains shown in SEQ ID NO.14 and the amino acid sequences of heavy chain shown in SEQ ID No.19, and with the DNA sequence of light chain shown in SEQ ID NO.4, and the DNA sequence of heavy chain shown in SEQ ID No.9.

Whereas, the de-immunized human anti-TNF-α antibody L1h3 with the amino acid sequence of light chains shown in SEQ ID NO.11 and the amino acid sequences of heavy chain shown in SEQ ID No.18, and with the DNA sequence of light chain shown in SEQ ID NO.1, and the DNA sequence of heavy chain shown in SEQ ID No.8.

Whereas, the de-immunized human anti-TNF-α antibody L2h1 with the amino acid sequence of light chains shown in SEQ ID NO.12 and the amino acid sequences of heavy chain shown in SEQ ID No.16, and with the DNA sequence of light chain shown in SEQ ID NO.2, and the DNA sequence of heavy chain shown in SEQ ID No.6.

Whereas, the de-immunized human anti-TNF-a antibody L2h5 with the amino acid sequence of light chains shown in SEQ ID NO.12 and the amino acid sequences of heavy chain shown in SEQ ID No.20, and with the DNA sequence of light chain shown in SEQ ID NO.2, and the DNA sequence of heavy chain shown in SEQ ID No.10.

Whereas, the de-immunized human anti-TNF-a antibody L0h4 with the amino acid sequence of light chains shown in SEQ ID NO.23 and the amino acid sequences of heavy chain shown in SEQ ID No.19, and with the DNA sequence of light chain shown in SEQ ID NO.21, and the DNA sequence of heavy chain shown in SEQ ID No.9.

The present invention features the expression plasmid containing the de-immunized anti-TNF-α antibody sequences of the present invention.

The present invention also covers the plasmid. Also disclosed is a host cell containing the de-immunnized anti- TNF-α antibody sequences.

The invention also provides de-immunized anti-TNF-a antibodies of the present invention for treatment of human diseases targeting TNF-α.

The TNF-α antibodies of the present invention can be used to inhibit the death of cells.

In addition, the TNF-α antibodies of the present invention can be used to treat human diseases including rheumatoid arthritis, Crohn's disease, psoriatic arthritis, and inflammatory bowel disease. These methods comprise administrating an effective amount of a TNF-α antibody of the present invention to a subject in need thereof.

Furthermore, the present invention also features pharmaceutical and diagnostic compositions comprising a TNF-α antibody of the present invention.

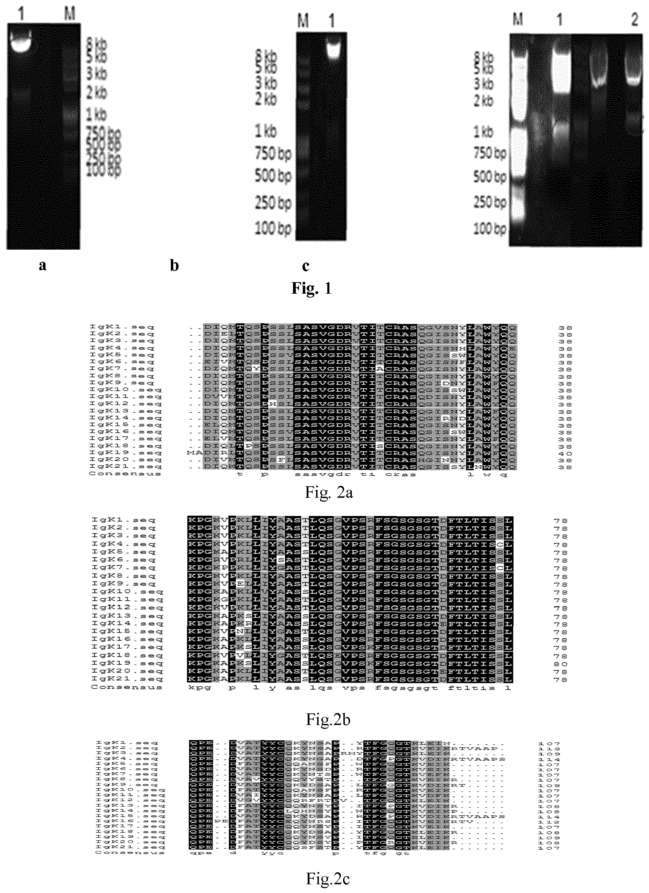

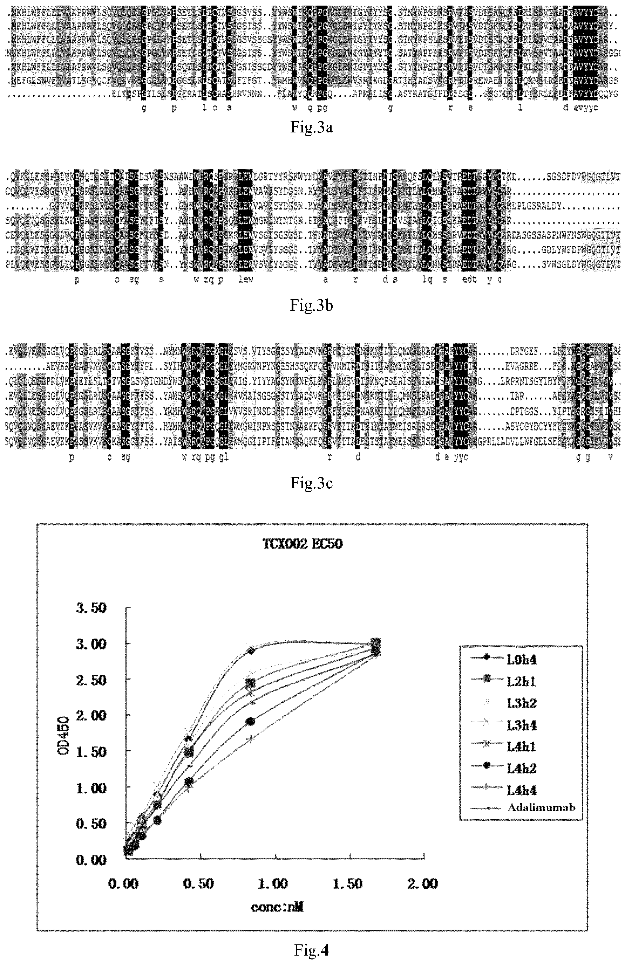

The present disclosure describes a method of de-immunogenisation of an anti-TNF-α monoclonal antibody, including:
1. Analysis the FR sequences of anti-TNF-α monoclonal antibody Adalimumab and identify the sequences with high immunogenicities.
2. Align the FR sequences of anti-TNF-α monoclonal antibody Adalimumab against the ones of human IgGs in NCBI database, and find the ones with high homologies but lower immunogenicities.
3. Replace the high immunogenic FR sequence(s) of anti-TNF-α monoclonal antibody Adalimumab with low immunogenic FR sequences from other human antibodies.
4. Perform 3D structure modeling of the newly designed antibody sequences against the anti-TNF-α monoclonal antibody Adalimumab using Pymol program to identify the ones with closest resembling of the original antibody.
5. Once the variable region sequences confirmed, chemically synthesize both the rariable sequences with artificially added restriction enzyme sites (Kpn I and BamH I for light chain variable region, KpnI and AgeI for heavy chain variable region), ligate to vector pJH16 to obtain the expression plasmids for heavy chain and light chain of human antibody (Results see Fig.1). Screen for positive clones after transformation by sequenceing and restriction enzyme digestions.
6. Extract the plasmids using the kit from Qiagen following the instruction from the manufacturer.
7. Transient co-transfect the different combinations of the human light and heavy chain expression plasmids produced different human anti-TNF-α monoclonal antibodies with different expression levels and affinities for TNF-α(see Fig.4).
8. Based on above data, a few combinations were selected to develop stable cell lines for over-expression of human anti-TNF-α.
9. The human anti-TNF-α monoclonal antibodies featured in this invention bind to the same antigenic eptiope as Adalimumab but with reduce immunogenicities and different 3D structures, longer half-lives, could be a better biotherapeutics.
10. The present invention features mthod to modify the immunogenicity of Adalimumab in human patients by replacing some of the amino acid sequences in Adalimumab with other human sequences.
11. The present invention provides examples to show the modified human anti-TNF-α monoclonal antibodies have similar affinities as Adalimumab but extend the half-lives with prolonger efficicacies.

### Legends:

Fig. 1 Digestions of Plasmids. 1-a, double digestions of pJH16 plasmid with Kpn I and Age I. 1-b, double digestions of pJH16 with Kpn I and BamH I. 1-c, lane 1, double digestions of the heavy chain of Adailimumab. Lane 2, double digestions of the light chain of Adalimumab. M, the DNA markers.
Fig. 2 Sequence blasts the light chains of the modified vs the one of Adalimumab.
Fig. 3 Sequence blasts the heavy chains of the modified vs the one of Adalimumab.
Fig. 4 The EC50s of modified human anti-TNF-α monoclonal antibodies.
Fig. 5 Inhibitions of TNF-α-induced cell toxicity in L929 cells by modified human anti-TNF-α monoclonal antibodies.
Fig. 6 PK study of modified human anti-TNF-α monoclonal antibodies.

### DETAILED DESCRIPTION

The present invention is as defined in the claims and relates to a low immunogenic human anti-TNF-α antibody comprising the CDR regions of heavy (SEQ ID NO. 23) and light (SEQ ID NO. 24) chains from human Adalimumab, but modification/replacement of amino acid sequence(s) in the FR regions of Adalimumab, wherein the antibody has reduced immunogenicity compared to Adalimumab, and wherein the antibody comprises:
(a) the human light chain amino acid sequence of SEQ ID NO: 13 and the human heavy chain amino acid sequence of SEQ ID NO: 17; or
(b) the human light chain amino acid sequence of SEQ ID NO: 13 and the human heavy chain amino acid sequence of SEQ ID NO: 19; or
(c) the human light chain amino acid sequence of SEQ ID NO: 14 and the human heavy chain amino acid sequence of SEQ ID NO: 16; or
(d) the human light chain amino acid sequence of SEQ ID NO: 14 and the human heavy chain amino acid sequence of SEQ ID NO: 17; or
(e) the human light chain amino acid sequence of SEQ ID NO: 14 and the human heavy chain amino acid sequence of SEQ ID NO: 19; or
(f) the human light chain amino acid sequence of SEQ ID NO: 12 and the human heavy chain amino acid sequence of SEQ ID NO: 16; or
(g) the human light chain amino acid sequence of SEQ ID NO: 23 and the human heavy chain amino acid sequence of SEQ ID NO: 19.

Accordingly, it should be understood that the following examples are given by way of illustration, not limitation.

Unless specified, all the techniques used are common practices and can be performed by skilled personel. All of the materials and reagents can be purchased commercially. To the extent that the examples include any subject-matter falling outside the scope of the claims, it is included merely for reference purposes.

### Example 1 Analysis and modification of the immunogenicity of the sequences Adalimumab

Used a program to examine the sequences of Adalimumab and found that the immunogenicity score is 16.

Used the same software to study the immunogenicities of the FRs of Adalimumab, identified the sequences with high immunogenicities, and searched human antibody sequence database for potential human sequences with lower immunogenicity.

Replaced the high immunogenic sequences in Adalimumab with the low immunogenic ones, and designed 5 human light chains L1-L5 (SEQ ID No.1-5) and 5 human heavy chains h1-h5 (SEQ ID No.6-10) for fully human anti-TNF-a monoclonal antibodies.

Perform 3D structure modeling of the newly designed antibody sequences against the ones of Adalimumab using Pymol program to identify the ones with closest resembling of the original antibody.

Fully human anti-TNF-a monoclonal antibodies can be any combination of one light chain from any one of L0-L5 (SEQ ID No.1-5, 21) and one heavy chain from any one of h1-h5 (SEQ ID No.6-10).

### Example 2 Construction of the expression plasmids of fully human anti-TNF-a monoclonal antibodies

Added the restriction sites of Kpn I and BamH I to the light chain variable region sequences and the restriction sites of Kpn I and Age I to the heavy chain variable region sequences obtained in Example 1. All the variable region of the light and heavy chain sequences were inserted into the plasmids. Cut the heavy chain variable region sequences from the plasmids and inserted into the corresponding sites of the expression vector pJH16 using the restriction sites of Kpn I and Age I. Cut the light chain variable region sequences from the vector and inserted into the corresponding sites of the expression vector pJH16 using the restriction sites of Kpn I and BamH I, to obtain the fully human monoclonal antibody heavy and light chain expression plasmids. The plasmids and the expression vectors were subjected to enzyme digestions at 37 C overnight. Results of digestions of light chain, heavy chain, and the expression vectors are shown in Figure 1. The bands of target genes and expression vectors were cut-out and extracted using Qiagen Gel Extraction Kit, then performed the ligations overnight using T4 DNA ligation system and transformed into E. coli DH5α. Colonies were picked for DNA sequencing and the alignments of sequencing data matched the designed gene 100%.

### Example 3 Transient Expression and purification of fully human anti-TNF-α monoclonal antibodies

Extracted the plasmids from the transformed E. coli DH5α, as shown in Example 2, using the Ultrapure Plasmid Prep kit from Qiagen.

Co-transfected the 293F cells with different combinations of the human light and heavy chain expression plasmids using lipofecting reagents from Invitogen. Total 31 combinations tried.

The expression levels of human IgGs in the culture supernants were examined on Day 3 and the expression levels ranged between 423.5∼2624ng/ml.

**Table 1 □ Expression Levels of Human Antibodies □ ng/ml □**

| Comb. | Conc. | Comb. | Conc. | Comb. | Conc. | Comb. | Conc. | Comb. | Conc. | Comb. | Conc. |
|---|---|---|---|---|---|---|---|---|---|---|---|
| L0h1 | 1530 | L1h1 | 1371 | L2hl | 1988 | L3hl | 2624 | L4hl | 810.7 | L5hl | 439.1 |
| L0h2 | 11172 | L1h2 | 487.6 | L2h2 | 755.8 | L3h2 | 1208 | L4h2 | 1130 | L5h2 | 423.5 |
| L0h3 | 2021 | L1h3 | 873.3 | L2h3 | 662.2 | L3h3 | 602.9 | L4h3 | 2206 | L5h3 | 797 |
| L0h4 | 1109 | L1h4 | 1257 | L2h4 | 476 | L3h4 | 1638 | L4h4 | 1381 | L5h4 | 475.9 |
| L0h5 | 1408 | L1h5 | 868 | L2h5 | 677.7 | L3h5 | 1282 | L4h5 | 1423 | L5h5 | 952.2 |
| Adh010 | 1892 | | | | | | | | | | |

| | | | | | | | | | | | |
|---|---|---|---|---|---|---|---|---|---|---|---|
| (Note: L0h1 means the combination of light chain L0 from Adalimumab and the heavy chain h1 from modified anti-TNF-a antibody) | | | | | | | | | | | |

Performed indirect ELISA against TNF-α coated on 96-well plate, and found some of them (L0h4, L3h4, L3h2, L4h4, etc) have strong signals as Adalimumab, and some of them lost the binding affinity (L0h2) (Data see Table 2)

**Table 2 ELISA Screening of Different Combinations against TNF-α**

| Comb. | L0h1 | | L0h2 | | L0h3 | | **L0h4** | | L0h5 | | NC | |
|---|---|---|---|---|---|---|---|---|---|---|---|---|
| OD | 2.158 | 2.182 | 0.057 | 0.054 | 0.68 | 0.768 | **3.339** | **3.133** | 1.03 | 0.873 | 0.09 | 0.059 |

| Comb. | L1h1 | | L1h2 | | **L1h3** | | Llh4 | | L1h5 | | NC | |
|---|---|---|---|---|---|---|---|---|---|---|---|---|
| OD | 1.926 | 2.401 | 2.268 | 2.459 | **1.413** | **1.431** | 2.621 | 2.552 | 0.824 | 1.051 | 0.045 | 0.057 |

| Comb. | **L2h1** | | L2h2 | | L2h3 | | L2h4 | | **L2h5** | | NC | |
|---|---|---|---|---|---|---|---|---|---|---|---|---|
| OD | **1.891** | **2.384** | 2.802 | 2.704 | 0.709 | 0.973 | 2.235 | 2.848 | **0.894** | **1.255** | 0.047 | 0.051 |

| Comb. | L3h1 | | **L3h2** | | L3h3 | | **L3h4** | | L3h5 | | L0h0 | |
|---|---|---|---|---|---|---|---|---|---|---|---|---|
| Comb. | 2.178 | 2.329 | **2.434** | **2.498** | 0.888 | 0.815 | **2.616** | **2.664** | 0.959 | 1.104 | 3.008 | 3.244 |

| OD | **L4h1** | | **L4h2** | | L4h3 | | **L4h4** | | L4h5 | | L0h0 | |
|---|---|---|---|---|---|---|---|---|---|---|---|---|
| | **1.978** | **2.182** | **2.968** | **2.546** | 1.617 | 1.607 | **2.904** | 2.757 | 0.714 | 0.972 | 2.877 | 3.041 |

| Comb. | L5hl | | **L5h2** | | L5h3 | | L5h4 | | L5h5 | | L0h0 | |
|---|---|---|---|---|---|---|---|---|---|---|---|---|
| OD | 0.864 | 1.583 | **1.857** | **1.821** | 1.366 | 1.404 | 1.528 | 1.643 | 0.885 | 0.903 | 2.926 | 3.172 |

### Example 4 Stable Expression and purification of fully human anti-TNF-a monoclonal antibodies

Based on above data, 10 combinations were selected to develop stable cell lines for over-expression of human anti-TNF-α.

CHO cells was electro-transfected and selected under MTX pressure (purchased from Sigma) in the selective Opti-CHO medium (purchased from Invitrogen). Five selecting gradients were set as 50nM, 100nM, 200nM, 400nM and 800nM. After each round, the expression levels of IgG in the culture supernatants on Day 7 were examined using Sandwich ELISA method. The results showed that stable expressions of IgGs were observed with all of the combinations but the levels were different (Table 3).

**Table 3 IgG Levels of different combinations at different stages**

| Comb. | opti-cho IgG (ng/ml) | 50nM IgG (ng/ml) | 100nM IgG (ng/ml) |
|---|---|---|---|
| adh010 | 30 | 134 | 346 |
| L3h2 | 92.7 | 122 | 237 |
| L3h4 | 87.4 | 251 | 367 |
| L5h2 | 30.8 | 129 | 452 |
| L4h1 | 104 | 176 | 258 |
| L4h2 | 127 | 318 | 523 |
| L4h4 | 72.5 | 939 | 734 |
| L1h3 | 97 | 160 | 270 |
| L2h1 | 64 | 208.6 | 471 |
| L0h4 | 30 | 389 | 598 |
| L2h5 | 29.2 | 226 | 476 |

When the process was complete, limiting dilution was performed for monoclonal cloning. Cells were seeded at 96-well plate and cultured at 37 °C 5% CO₂. 14 days later, 50 µl of supernatant was collected for antibody production testing using sandwich ELISA method. Clones with higher expressing levels were selected for further expansion.

Used a Protein-A affinity chromatography column to purify the human anti-TNF-α antibodies from the culture supernatants of the 11 stable cell lines. The concentrations of antibodies were determined by OD280/1.4. The purities of the antibodies were examined by SDS-PAGE analysis.

### Example 5 Biological activities of human anti-TNF-a antibodies

1. Affinities: The EC50s of the newly invented human anti-TNF-a antibodies were compared with the one of Adalimumab using Indirect ELISA. The wells of 96-well plates were coated with 300ng/ml of TNF-α in PBS overnight at 4 C. After wash, the wells were blocked with 5% skim milk in PBS for 1 hour at room temperature. Various concentrations of antibodies diluted in 5% skim milk-PBS were added to the wells and incubated for 1 hour at room temperature. After another wash, HRP-conjugated goat-anti-human IgG secondary antibodies were added and incubated for another 1 hour. After through wash, the substrates were added and the absorbances at 450 nm were measured. As shown in Table 4, some of the newly invented human anti-TNF-a antibodies have very similar EC50 as Adalimumab.

**Table 4 EC50s of human anti-TNF-a antibodies**

| Comb. | 10h4 | 12h1 | 13h2 | 13h4 | 14h1 | 14h2 | 14h4 | adh010 |
|---|---|---|---|---|---|---|---|---|
| EC50(nM) | 0.37 | 0.43 | 0.40 | 0.34 | 0.47 | 0.60 | 0.69 | 0.49 |

2. Specificities: The specificities of the newly invented human anti-TNF-a antibodies were examined by Indirect ELISA against TNF-a and other cytokines. The wells of 96-well plates were coated with 1000ng/ml of rhTNFα, rhTNFβ, rIFNγ, IL-1α, IL-1β, IL-2, IL-4 and IL-8 in PBS overnight at 4 C. After wash, the wells were blocked with 5% skim milk in PBS for 1 hour at room temperature. Different human anti-TNF-a antibodies diluted in 5% skim milk-PBS were added to the wells and incubated for 1 hour at room temperature. After another wash, HRP-conjugated goat-anti-human IgG secondary antibodies were added and incubated for another 1 hour. After through wash, the substrates were added and the absorbances at 450 nm were measured. As shown in Table 5, all of the newly invented human anti-TNF-a antibodies are very specific for TNF-α. 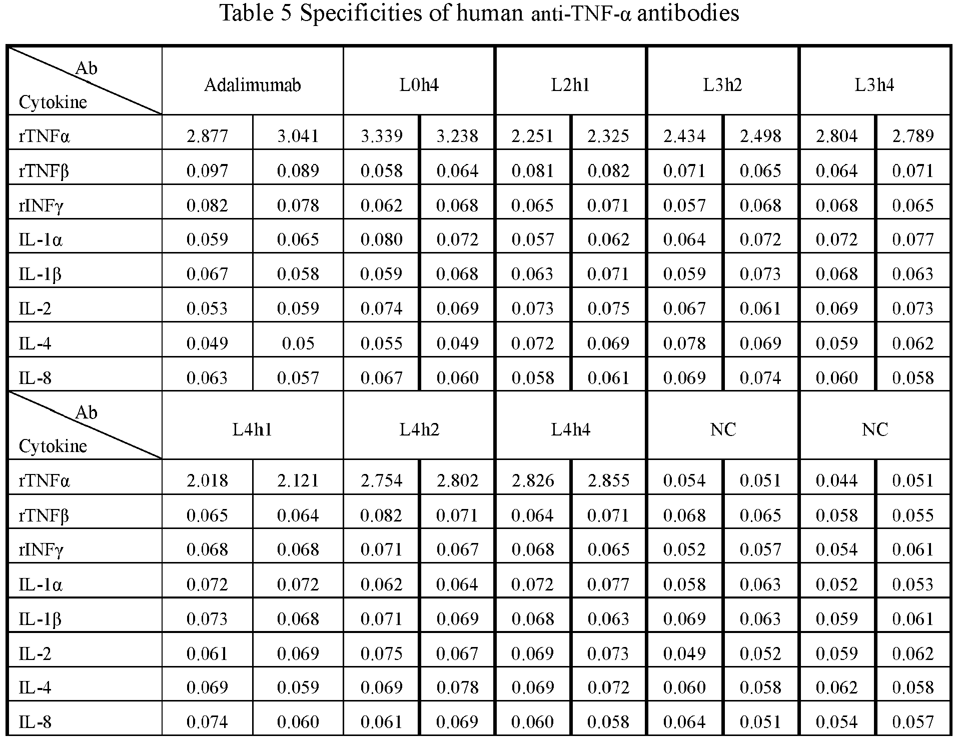
3. Inhibition of TNF-α induced apotosis.

L929 cells were seeded at 50,000 cells/well of 96-well plate in RPMI-1640-10% FBS and incubated at 37 °C 5% CO2. 4 hours later, discard the medium and added 100 µl/well of different concentrations of ADALIMUMAB or the invented human anti-TNF-a antibodies in RPMI-1640-10% FBS plus Actinomysin D 1ug/ml at 37 °C 5% CO2. One day's later, the cell numbers in each well were determined by CKK assay.

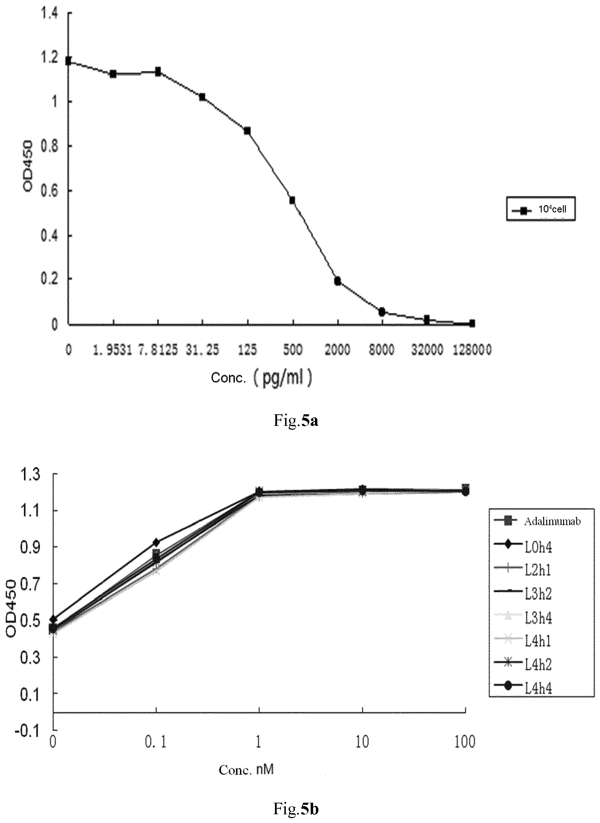

As shown in Figure 5, both ADALIMUMAB and the newly invented human anti-TNF-α antibodies could inhibit TNF-α induced apoptosis of L929 cells.

### Example 6 Immunogenicity and PK in mice

1. Immunogenicity: Mice were injected with all 10 new human anti-TNF-α antibodies and Adalimumab with the adjuvent. 14 days' later, the tail bleeds were examined by ELISA against their antigens respectively. As shown in Table 6, the anti-drug antibody titers of some newly invented human anti-TNF-a antibodies were at least 5-time lower than the one of Adalimumab.

**Table 6 ADA Titers of human anti-TNF-α antibodies in mice**

| Titers | | 1:500 | 1:1000 | 1:5000 | 1:10000 | 1:50000 | NC |
|---|---|---|---|---|---|---|---|
| | L3h2 | 0.974 | 0.459 | 0.056 | 0.064 | 0.051 | 0.042 |
| | L3h4 | 0.676 | 0.385 | 0.044 | 0.043 | 0.046 | 0.046 |
| | L5h2 | 0.854 | 0.435 | 0.042 | 0.047 | 0.047 | 0.047 |
| | L4h1 | 0.699 | 0.311 | 0.054 | 0.058 | 0.047 | 0.045 |
| | L4h2 | 1.207 | 0.607 | 0.062 | 0.049 | 0.042 | 0.042 |
| Comb. | L4h4 | 0.713 | 0.379 | 0.059 | 0.048 | 0.048 | 0.047 |
| | L0h4 | 1.016 | 0.591 | 0.048 | 0.067 | 0.054 | 0.056 |
| | Llh3 | 1.156 | 0.548 | 0.043 | 0.080 | 0.053 | 0.055 |
| | L2h1 | 0.781 | 0.389 | 0.041 | 0.056 | 0.059 | 0.057 |
| | L2h5 | 0.802 | 0.410 | 0.032 | 0.066 | 0.053 | 0.047 |
| | L0h0 | 2.614 | 1.311 | 0.2614 | 0.144 | 0.131 | 0.144 |

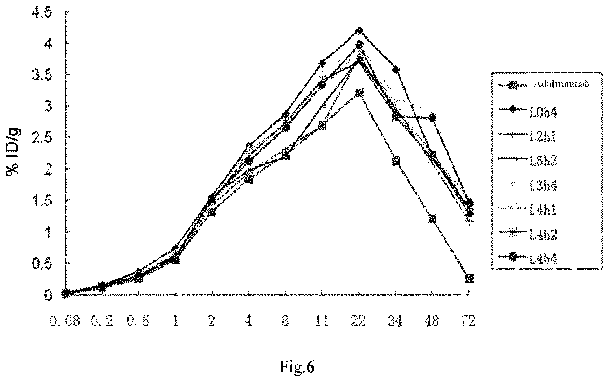

2. Pharmakintics: Mice were tail vent-injected with 125 I -labeled all 10 new human anti-TNF-α antibodies and Adalimumab (370 kBq , 2µg), 5 mice per group. At various time points (5, 12, 30 min, 1, 2, 4, 8, 11, 22, 34, 48, 72 h), the blood samples were collected and the radioactivities were measured. As shown in Fig 6, the PK of newly invented human anti-TNF-a antibodies were similar or better than the one of Adalimumab.

### Industrial Applications

The invention features human anti-TNF-α antibodies which share the CDRs of the amino acid sequences from Adalimumab but with different FRs from other human IgGs. The newly invented human anti-TNF-α antibodies have the same specificities, similar affinities and inhibitory activities against TNF-a but much lower immunogenicities than Adalimumab. The disclosure also features method of de-immunogenicity of human antibodies by replacing the high immunogenic FR sequences with lower ones from other human IgGs without altering the activities of the antibody significantly. Reduced immunogenicity will significantly reduce the level of anti-drug antibody in the patients treated with anti-TNF-a drug, extend drug's half-life and increase the efficacy of the biological drugs.

## Claims

1. A human anti-TNF-α antibody comprising the CDR regions of heavy (SEQ ID NO.23) and light (SEQ ID NO.24) chains from human Adalimumab, but modification/replacement of amino acid sequence(s) in the FR regions of Adalimumab, wherein the antibody has reduced immunogenicity compared to Adalimumab, and wherein the antibody comprises:
(a) the human light chain amino acid sequence of SEQ ID NO: 13 and the human heavy chain amino acid sequence of SEQ ID NO: 17; or
(b) the human light chain amino acid sequence of SEQ ID NO: 13 and the human heavy chain amino acid sequence of SEQ ID NO: 19; or
(c) the human light chain amino acid sequence of SEQ ID NO: 14 and the human heavy chain amino acid sequence of SEQ ID NO: 16; or
(d) the human light chain amino acid sequence of SEQ ID NO: 14 and the human heavy chain amino acid sequence of SEQ ID NO: 17; or
(e) the human light chain amino acid sequence of SEQ ID NO: 14 and the human heavy chain amino acid sequence of SEQ ID NO: 19; or
(f) the human light chain amino acid sequence of SEQ ID NO: 12 and the human heavy chain amino acid sequence of SEQ ID NO: 16; or
(g) the human light chain amino acid sequence of SEQ ID NO: 23 and the human heavy chain amino acid sequence of SEQ ID NO: 19.

2. The low immunogenic human anti-TNF-α antibody of Claim 1, comprising the human light chain amino acid sequence of SEQ ID NO. 13 and the human heavy chain amino acid sequence of SEQ ID NO. 19.

3. A DNA encoding the antibody of Claim 1 or 2.

4. An expression vector containing the DNA of Claim 3.

5. The DNA of Claim 3 or the expression vector of Claim 4 for use as a medicament.

6. The antibody of Claim 1 or 2 for use as a medicament.

7. The antibody of Claim 1 or 2 for use in treating a human autoimmune or inflammation disease.

8. The antibody for the use of Claim 7, wherein the disease is rheumatoid arthritis or lupus erythematosus.

9. The antibody of Claim 1 or 2 for use in a diagnostic application.

## Patentansprüche

1. Ein menschlicher Anti-TNF-a-Antikörper, der die CDR-Regionen von schwer umfasst (SEQ ID NO.23) und leichte (SEQ ID NO.24) Ketten von menschlichem Adalimumab, aber Modifikation / Ersatz von Aminosäuresequenz (en) in den FR-Regionen von Adalimumab, wobei der Antikörper im Vergleich zu Adalimumab eine verringerte Immunogenität aufweist und wobei der Antikörper umfasst:
(a) die Aminosäuresequenz der menschlichen leichten Kette von SEQ ID NO: 13 und die Aminosäuresequenz der menschlichen schweren Kette von SEQ ID NO: 17; oder
(b) die Aminosäuresequenz der menschlichen leichten Kette von SEQ ID NO: 13 und die Aminosäuresequenz der menschlichen schweren Kette von SEQ ID NO: 19; oder
(c) die Aminosäuresequenz der menschlichen leichten Kette von SEQ ID NO: 14 und die Aminosäuresequenz der menschlichen schweren Kette von SEQ ID NO: 16; oder
(d) die Aminosäuresequenz der menschlichen leichten Kette von SEQ ID NO: 14 und die Aminosäuresequenz der menschlichen schweren Kette von SEQ ID NO: 17; oder
(e) die Aminosäuresequenz der menschlichen leichten Kette von SEQ ID NO: 14 und die Aminosäuresequenz der menschlichen schweren Kette von SEQ ID NO: 19; oder
(f) die Aminosäuresequenz der menschlichen leichten Kette von SEQ ID NO: 12 und die Aminosäuresequenz der menschlichen schweren Kette von SEQ ID NO: 16; oder
(g) die Aminosäuresequenz der menschlichen leichten Kette von SEQ ID NO: 23 und die Aminosäuresequenz der menschlichen schweren Kette von SEQ ID NO: 19.

2. Niedrig immunogener menschlicher Anti-TNF-a-Antikörper nach Anspruch 1, umfassend die Aminosäuresequenz der menschlichen leichten Kette von SEQ ID NO. 13 und die Aminosäuresequenz der menschlichen schweren Kette von SEQ ID NO. 19.

3. DNA, die den Antikörper nach Anspruch 1 oder 2 codiert.

4. Expressionsvektor, der die DNA von Anspruch 3 enthält.

5. DNA nach Anspruch 3 oder Expressionsvektor nach Anspruch 4 zur Verwendung als Medikament.

6. Antikörper nach Anspruch 1 oder 2 zur Verwendung als Medikament.

7. Antikörper nach Anspruch 1 oder 2 zur Verwendung bei der Behandlung einer menschlichen Autoimmunerkrankung oder Entzündung Krankheit.

8. Antikörper zur Verwendung nach Anspruch 7, wobei die Krankheit rheumatoide Arthritis oder Lupus erythematodes ist.

9. Antikörper nach Anspruch 1 oder 2 zur Verwendung in einer diagnostischen Anwendung.

## Revendications

1. Anticorps anti-TNF-a humain comprenant les régions CDR des chaînes lourdes (SEQ ID NO.23) et légères (SEQ ID NO.24) de l'adalimumab humain, mais modification / remplacement de la ou des séquences d'acides aminés Régions FR de l'adalimumab, dans lesquelles l'anticorps a une immunogénicité réduite par rapport à l'adalimumab, et dans lesquelles l'anticorps comprend:
(a) la séquence d'acides aminés de chaîne légère humaine de SEQ ID NO: 13 et la séquence d'acides aminés de chaîne lourde humaine de SEQ ID NO: 17; ou
(b) la séquence d'acides aminés de chaîne légère humaine de SEQ ID NO: 13 et la séquence d'acides aminés de chaîne lourde humaine de SEQ ID NO: 19; ou
(c) la séquence d'acides aminés de chaîne légère humaine de SEQ ID NO: 14 et la séquence d'acides aminés de chaîne lourde humaine de SEQ ID NO: 16; ou
(d) la séquence d'acides aminés de chaîne légère humaine de SEQ ID NO: 14 et la séquence d'acides aminés de chaîne lourde humaine de SEQ ID NO: 17; ou
(e) la séquence d'acides aminés de chaîne légère humaine de SEQ ID NO: 14 et la séquence d'acides aminés de chaîne lourde humaine de SEQ ID NO: 19; ou
(f) la séquence d'acides aminés de chaîne légère humaine de SEQ ID NO: 12 et la séquence d'acides aminés de chaîne lourde humaine de SEQ ID NO: 16; ou
(g) la séquence d'acides aminés de chaîne légère humaine de SEQ ID NO: 23 et la séquence d'acides aminés de chaîne lourde humaine de SEQ ID NO: 19.

2. Anticorps anti-TNF-a humain faiblement immunogène selon la revendication 1, comprenant la séquence d'acides aminés de la chaîne légère humaine de SEQ ID NO. 13 et la séquence d'acides aminés de la chaîne lourde humaine de SEQ ID NO. 19.

3. Un ADN codant pour l'anticorps de la revendication 1 ou 2.

4. Vecteur d'expression contenant l'ADN de la revendication 3.

5. ADN selon la revendication 3 ou vecteur d'expression selon la revendication 4 pour une utilisation comme médicament.

6. Anticorps selon la revendication 1 ou 2 à utiliser comme médicament.

7. Anticorps selon la revendication 1 ou 2 destiné à être utilisé dans le traitement d'un auto-immun ou d'une inflammation humaine.
maladie.

8. Anticorps pour l'utilisation selon la revendication 7, dans lequel la maladie est la polyarthrite rhumatoïde ou le lupus érythémateux.

9. Anticorps selon la revendication 1 ou 2 à utiliser dans une application de diagnostic.
